# EUROPEAN PATENT APPLICATION

(11) **EP 4 545 115 A1**
(43) Date of publication of application: **30.04.2025**
(21) Application number: 24205552.3
(22) Date of filing: 09.10.2024
(51) Int. Cl.: A61M 1/36

(54) **EXTRACORPOREAL LIFE SUPPORT SYSTEM WITH BLOCKAGE DETECTION**

(30) Priority: 25.10.2023 US 202318493959
(71) Applicant: LivaNova Deutschland GmbH, 80939 Munich (DE)
(72) Inventor: ARTELSMAIR, Andreas, 81739 München (DE); KOHNEN, Lena, 81369 München (DE); HERZOG, Helen, 85748 Garching bei München (DE)
(74) Representative: Grünecker Patent- und Rechtsanwälte PartG mbB

(57) **Abstract**

An blood treatment system including blockage detection capabilities. The system may include a sensor positioned along a blood pathway and a control unit in communication therewith. The sensor may sense a parameter of blood passing through the blood pathway and may automatically transmit a first signal corresponding to the parameter to the control unit. Further, the control unit may receive the first signal and automatically transmit a second signal to a control element and the control element may receive the second signal from the control unit. Further, the control element may reduce the blood flow within the venous blood pathway in response to receiving the second signal, and wherein reducing the blood flow substantially equalizes a blood pressure within the lumen of the distal end region of a cannula and the blood pressure of a region of the venous blood pathway adjacent to the distal end region of the cannula.

## Description

### TECHNICAL FIELD

The present disclosure relates to an extracorporeal life support system and methods for manufacturing and/or using an extracorporeal life support system. More particularly, the present disclosure relates to an extracorporeal life support system including blockage detection capabilities and methods for manufacturing and/or using an extracorporeal life support system.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by an extracorporeal perfusion system. An extracorporeal perfusion system may provide both cardiac and respiratory support to a patient whose heart and lungs are unable to provide an adequate amount of gas exchange during a cardiac and pulmonary procedure. Extracorporeal perfusion works by removing blood from a patient's body to oxygenate the red blood cells while also removing carbon dioxide. The oxygenated blood is then returned to the patient.

Extracorporeal perfusion systems may include multiple devices that together form a blood recirculation loop between the patient and a blood oxygenator. For example, some extracorporeal perfusion systems may include a blood reservoir, a blood pump to power blood flow, an oxygenator to oxygenate the blood, a device to filter the blood (which may be included within the oxygenator and/or the reservoir in some systems), one or more sensors positioned at various locations along blood pathways and one or more control units. It can be appreciated that a blood pathway (e.g., tubing) may extend from the patient to the blood reservoir, then towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

It can be further appreciated that removing the blood from the patient may include placing multiple cannulae into the patient's body in a variety of locations, depending on the type of medical procedure being performed. For example, to infuse oxygen-rich blood into the patient's body, an arterial cannula may be placed in the ascending aorta of the patient. Additionally, to remove deoxygenated blood from the patient's body, a venous cannula may be placed in the superior vena cava and/or inferior vena cava of the patient. Blood exiting the patient's body may enter the venous cannula due to a negative pressure in the venous line. The negative pressure may be created in a variety of ways, including via gravitation, a kinetic assist device (e.g., pump) and/or a vacuum module. However, in some instances, the suction created by the negative pressure may force the vessel wall to collapse onto the outer surface of the cannula, thereby blocking the apertures which allow blood to flow therethrough and effectively blocking the flow of blood within the blood pathway. Therefore, it may be desirable to design an extracorporeal perfusion system which may include one or more cannula blockage detection systems configured to monitor the flow of blood within the extracorporeal perfusion system and automatically unblock a blocked cannula. Extracorporeal perfusion systems including cannula blockage detection systems configured to monitor the flow of blood within the extracorporeal perfusion system and alert a user to a potential blockage and/or automatically unblock a blocked cannula are disclosed herein.

### SUMMARY

An example extracorporeal blood treatment system may include a cannula positioned within a venous blood pathway extending between the patient and a reservoir, wherein the cannula includes a distal end region and a lumen extending therein. The system may also include a first sensor positioned along the venous blood pathway and a control unit in communication with the first sensor. Further, the first sensor is configured to sense a first sensor parameter of blood passing through the venous blood pathway and the first sensor is configured to automatically transmit a first signal corresponding to the first sensor parameter to the control unit. Further, the control unit is configured to receive the first signal and automatically transmit a second signal to a first control element and the first control element is configured to receive the second signal from the control unit. Further, the first control element is configured to reduce the blood flow within the venous blood pathway in response to receiving the second signal, and wherein reducing the blood flow substantially equalizes a blood pressure within the lumen of the distal end region of the cannula and the blood pressure of a region of the venous blood pathway adjacent to the distal end region of the cannula.

In addition or alternatively to any example described herein, the first sensor is a blood flow sensor.

In addition or alternatively to any example described herein, the first sensor parameter is a flowrate of blood passing through the venous blood pathway.

In addition or alternatively to any example described herein, the first sensor is a blood pressure sensor.

In addition or alternatively to any example described herein, the first sensor parameter is a pressure of blood passing through the venous blood pathway, the reservoir or both the venous blood pathway and the reservoir.

In addition or alternatively to any example described herein, the first control element includes a clamp, and wherein the clamp is configured to decrease the flowrate of blood flowing through the venous blood pathway in response to receiving the second signal from the control unit.

In addition or alternatively to any example described herein, the control unit is configured to automatically actuate the clamp in response to receiving the first signal from the first sensor.

In addition or alternatively to any example described herein, the first control element includes a pump, and wherein the pump is configured to adjust the flowrate of blood flowing through the venous blood pathway in response to receiving the second signal from the control unit.

In addition or alternatively to any example described herein, the control unit is configured to automatically decrease a speed of the pump in response to receiving the first signal from the first sensor.

In addition or alternatively to any example described herein, the first control element includes a vacuum unit, and wherein the vacuum unit is configured to adjust the flowrate of blood flowing through the venous blood pathway in response to receiving the second signal from the control unit.

In addition or alternatively to any example described herein, the control unit is configured to automatically reduce the suction of the vacuum unit in response to receiving the first signal from the first sensor.

In addition or alternatively to any example described herein, equalizing the blood pressure within the lumen of the distal end region of the cannula and the blood pressure of a region of the venous blood pathway adjacent to the distal end region of the cannula restores blood flow within the lumen of the cannula.

Another example extracorporeal blood treatment system includes a cannula positioned within a venous blood pathway extending between the patient and a reservoir, wherein the cannula includes a distal end region and a lumen extending therein. The blood treatment system also includes a first sensor positioned along the venous blood pathway, a second sensor positioned along the venous blood pathway and a control unit in communication with the first sensor and the second sensor. Further, the first sensor is configured to sense a first sensor parameter of blood passing through the venous blood pathway, the second sensor is configured to sense a second sensor parameter of blood passing through the venous blood pathway, the first sensor is configured to transmit a first signal corresponding to the first sensor parameter to the control unit, the second sensor is configured to transmit a second signal corresponding to the second sensor parameter to the control unit, the control unit is configured to receive the first signal and transmit a third signal to a first control element, the control unit is configured to receive the second signal and transmit a fourth signal to the first control element and the first control element is configured to receive the third signal and the fourth signal from the control unit. Further, the first control element is configured to reduce the blood flow within the venous blood pathway in response to receiving the third signal, the fourth signal or both the third and the fourth signals, and wherein reducing the blood flow substantially equalizes a blood pressure within the lumen of the distal end region of the cannula and the blood pressure of a region of the venous blood pathway adjacent to the distal end region of the cannula.

In addition or alternatively to any example described herein, the first sensor is a blood flow sensor and the second sensor is a blood pressure sensor.

In addition or alternatively to any example described herein, the first sensor parameter is a flowrate of blood passing through the venous blood pathway and the second sensor parameter is a pressure of blood passing through the venous blood pathway.

In addition or alternatively to any example described herein, the first control element includes a clamp, and wherein the clamp is configured to automatically adjust the flowrate of blood flowing through the venous blood pathway in response to receiving the third signal, the fourth signal or both the third and the fourth signals.

In addition or alternatively to any example described herein, the first control element includes a pump, and wherein the pump is configured to automatically change the flowrate of blood flowing through the venous blood pathway in response to receiving the third signal, the fourth signal or both the third and the fourth signals.

In addition or alternatively to any example described herein, the first control element includes a vacuum unit, and wherein the vacuum unit is configured to change the flowrate of blood flowing through the venous blood pathway in response to receiving the third signal, the fourth signal or both the third and the fourth signals.

Another example extracorporeal blood treatment system includes a first control element coupled to a venous blood pathway extending between a patient and a reservoir, a blood flowrate sensor positioned along the venous blood pathway, a blood pressure sensor positioned along the venous blood pathway and a control unit in communication with the blood flowrate sensor, the blood pressure sensor, and the first control element. Further, the blood flowrate sensor is configured to automatically transmit a first signal to the control unit, wherein the first signal corresponds to a flowrate of blood in the venous blood pathway, the blood pressure sensor is configured to automatically transmit a second signal to the control unit, wherein the second signal corresponds to a pressure of blood in the venous blood pathway, the control unit is configured to automatically receive the first signal and the second signal and the control unit is configured to automatically send a third signal to the first control element in response to receiving the first signal, the second signal or both the first and the second signals. Further, the first control element is configured to automatically reduce the blood flow within the venous blood pathway in response to receiving the third signal, and wherein reducing the blood flow substantially equalizes a blood pressure within the lumen of the distal end region of the cannula and the blood pressure of a region of the venous blood pathway adjacent to the distal end region of the cannula.

In addition or alternatively to any example described herein, wherein equalizing the blood pressure within the lumen of the distal end region of the cannula and the blood pressure of a region of the venous blood pathway adjacent to the distal end region of the cannula restores blood flow within the lumen of the cannula.

In addition or alternatively to any example described herein, wherein the first control element includes a clamp, and wherein the clamp is configured to automatically adjust the flowrate of blood flowing through the venous blood pathway in response to receiving the third signal, the fourth signal or both the third and the fourth signals.

In addition or alternatively to any example described herein, wherein the first control element includes a pump, and wherein the pump is configured to automatically change the flowrate of blood flowing through the venous blood pathway in response to receiving the third signal, the fourth signal or both the third and the fourth signals.

In addition or alternatively to any example described herein, wherein the first control element includes a vacuum unit, and wherein the vacuum unit is configured to change the flowrate of blood flowing through the venous blood pathway in response to receiving the third signal, the fourth signal or both the third and the fourth signals.

Another example extracorporeal blood treatment system includes a cannula positioned within a venous blood pathway extending between the patient and a reservoir, wherein the cannula includes a distal end region and a lumen extending therein, a first sensor positioned along the venous blood pathway and a control unit in communication with the first sensor. Further, the first sensor is configured to sense a first sensor parameter of blood passing through the venous blood pathway, the first sensor is configured to automatically transmit a first signal corresponding to the first sensor parameter to the control unit, the control unit is configured to receive the first signal and automatically transmit a second signal to one or more of a clamp, a pump and a vacuum unit. Further, one or more of the clamp, the pump and the vacuum unit are configured to receive the second signal from the control unit, one or more of the clamp, the pump and the vacuum unit are configured to reduce the blood flow within the venous blood pathway in response to receiving the second signal, and wherein reducing the blood flow substantially equalizes a blood pressure within the lumen of the distal end region of the cannula and the blood pressure of a region of the venous blood pathway adjacent to the distal end region of the cannula.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates an example extracorporeal blood treatment system;
FIG. 2 is a schematic diagram of a computing device;
FIG. 3 illustrates a cannula positioned within a blood pathway;
FIG. 4 illustrates blood flowing from a blood pathway into and through the lumen of the cannula shown in FIG. 3;
FIG. 5 illustrates a blockage of the cannula shown in FIGS. 3-4;
FIG. 6 illustrates another example extracorporeal blood treatment system;
FIG. 7 illustrates another example extracorporeal blood treatment system.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5). As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen and releases carbon dioxide. After absorbing oxygen and releasing carbon dioxide in the lungs, the blood becomes oxygenated arterial blood. The oxygenated blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood and/or removing carbon dioxide, an oxygenator located outside the body may be used to oxygenate the blood and/or remove carbon dioxide. As discussed above, extracorporeal perfusion is a breathing and heart pumping life support system that may be utilized to support patients while medical treatments (e.g., heart surgery) are performed to treat their underlying illness. When supported via an extracorporeal perfusion system, oxygenation of the patient's blood and removal of carbon dioxide may occur outside the body.

Extracorporeal perfusion is generally performed using a heart-lung bypass system, which may be referred to as a "circuit." The circuit may include a blood flowpath exterior of the patient, such as one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

In some examples, an extracorporeal perfusion circuit may include a blood pump, oxygenator, tubing pathways (for transfer to and from the body), sensors (e.g., flow, pressure, bubble, temperature, oxygen, carbon dioxide, etc.), a heat exchanger (to cool and/or heat the blood), a control unit, and arterial and/or venous access points for the collection of blood in the circuit. It can be appreciated that the function of the blood pump is to generate blood flow within the extracorporeal perfusion circuit (e.g., circulate blood from the patient to the oxygenator and back to the patient) and to also generate blood pressure within the patient's vascular system. The blood pump may be positioned in the tubing pathway between the patient and the oxygenator. In some extracorporeal perfusion systems a roller pump may be utilized to generate blood flow within the extracorporeal perfusion circuit. However, in other extracorporeal perfusion systems, other blood pumps, including centrifugal pumps may be utilized to generate blood flow within the extracorporeal perfusion circuit.

In some extracorporeal perfusion systems, the oxygenator may include a housing having multiple chambers or pathways separated by a semi-permeable membrane, whereby the patient's blood may flow through one chamber or pathway, while an oxygen gas mixture (i.e., sweep gas) flows through another chamber or pathway. The semi-permeable membrane may include multiple microporous hollow fibers, each fiber having a lumen extending therethrough through which the oxygen gas mixture flows. The gas exchange may occur via diffusion of the gases across multiple microporous fibers, whereby oxygen moves from the inside of the hollow fibers into the blood while carbon dioxide diffuses from the blood into the interior of the hollow fibers, where it is swept away by the sweep gas flowing through the fiber. This gas exchange allows for oxygenation of venous blood and removal of carbon dioxide. In some extracorporeal perfusion systems, the oxygenator may include integrated heat exchangers that allow circulating blood to be cooled and/or warmed prior to returning to the patient.

In some instances, it may be desirable to control one or more parameters of blood flow through the various blood pathways of the extracorporeal perfusion system. For example, it may be desirable to control the flowrate of blood within one or more blood pathways within the extracorporeal perfusion system. In some examples, the flowrate of blood within the blood pathways of the extracorporeal perfusion system may be controlled via a combination of clamps, sensors and pumps, one or more of which may be coupled to the various blood pathways of the extracorporeal perfusion system. In some examples, one or more of the clamps, sensors and pumps may be in communication (e.g., wireless, wired communication, or other communication means capable of transmitting signals) with a control unit, whereby the control unit may be configured to operate one or more of the clamps, sensors and pumps in various combinations to control the flow of blood within the blood pathways of the extracorporeal perfusion system.

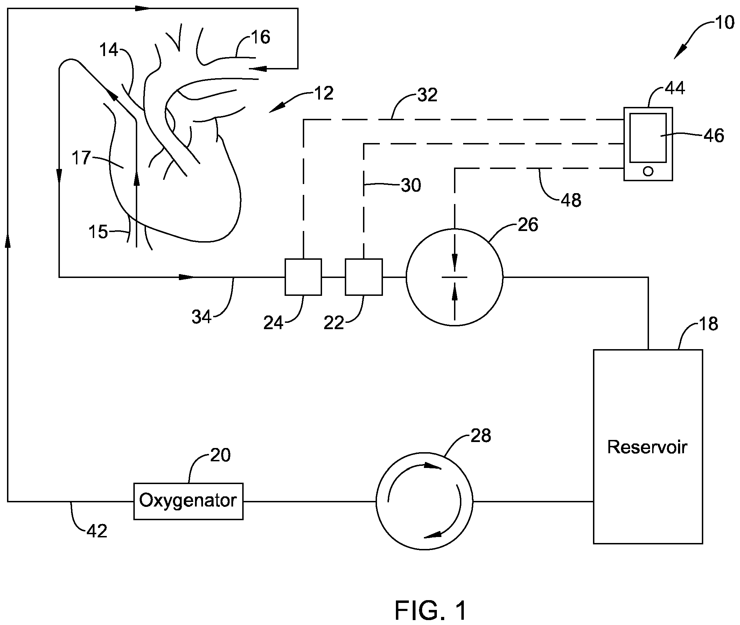

FIG. 1 illustrates an extracorporeal perfusion system 10. The extracorporeal perfusion system 10 may include a blood reservoir 18, an oxygenator 20, clamp 26, flow sensor 22, pressure sensor 24, pump 28 (e.g., roller pump) and a control unit 44. In some examples, the pressure sensor 24 may be positioned adjacent to or integrated with the blood reservoir 18. Additionally, the extracorporeal perfusion system 10 may include one or more blood pathways extending between various components of the extracorporeal perfusion system 10.

The blood reservoir 18 of the extracorporeal perfusion system 10 may be designed to hold blood which is gravity fed from a patient, such as the patient's superior vena cava (SVC) 14 and inferior vena cava (IVC) 15 or, alternatively, from a single cannula placed in the patient's right atrium 17. Generally, blood from the reservoir 18 may then pass to a pump 28 along a blood pathway 42. The pump 28 may then pump the blood into an oxygenator 20 along the blood pathway 42. After gas exchange takes place within the semi-permeable membrane of the oxygenator 20, the oxygenated blood may return to the arterial system of the patient, such as via a cannula placed in the aorta 16.

In some examples, the blood reservoir 18 and the oxygenator 20 may be coupled together in a variety of configurations. For example, the oxygenator 20 and the blood reservoir 18 may be combined into a single unit. In yet other examples, the blood reservoir 18 and the oxygenator 20 may be separate components within the extracorporeal perfusion system 10.

As discussed herein, the extracorporeal perfusion system 10 shown in FIG. 1 may also include a clamp 26, the flow sensor 22 and the pressure sensor 24 positioned along various blood pathways (e.g., venous and arterial blood pathways), whereby the clamp 26 and sensors 22, 24 may help regulate the flow of blood through the blood pathways. For example, FIG. 1 illustrates that the extracorporeal perfusion system 10 may include a clamp 26, the flow sensor 22 and the pressure sensor 24 positioned along the venous blood pathway 34. The arterial return blood pathway 42 may be defined as the blood pathway 42 along which oxygenated blood exiting the oxygenator 20 flows back to the aorta 16 of the patient.

When coupled to the venous blood pathway 34, the clamp 26 may be configured to actuate such that the clamp 26 decreases or increases the cross-sectional area of a component defining the venous blood pathway 34. For example, the venous blood pathway 34 may be formed from a tubing having a wall and a lumen extending therein. The lumen of the tubing may have a cross-sectional area which, along with the velocity of the blood flowing through the tubing, defines the volume of blood which may pass through the tubing over a given time period.

In some examples, the tubing used to define the venous blood pathway 34 may be formed from a polymer tubing (e.g., polyvinyl tubing). For example, the tubing used to define the venous blood pathway 34 may be constructed from polyvinyl chloride (PVC) because it is flexible, compatible with blood, inert, nontoxic, smooth, tough, transparent, resistant to kinking and collapse, and may be heat sterilized.

In other examples, the venous blood pathway 34 may be formed from other structures and materials. Additionally, some alternative materials that may be utilized to form the venous blood pathway 34 may include silicone. For example, the venous blood pathway 34, or portions thereof, may be formed from rigid tubing components having a lumen extending therethrough.

As discussed herein, when positioned along the blood pathway 34, the clamp 26 may be configured to actuate such that the clamp 26 decreases or increases the cross-sectional area of a lumen of a component defining the venous blood pathway 34. In some examples, the clamp 26 may engage tubing defining the blood pathway 34. In these examples, the tubing defining the blood pathway 34 may extend within at least a portion of the clamp 26, whereby actuation of the clamp 26 may either clamp down and restrict the cross-sectional area of the tubing or may release and expand the cross-sectional area of the tubing defining the blood pathway 34. In other words, the clamp 26 may be designed to physically deform the tubing to adjust the cross-sectional area of the lumen (which may, in turn, increase the resistance of the tubing), and therefore, the flowrate of blood through the tubing.

In other examples, the clamp 26 may include a component (e.g., a ball valve, iris, etc.) having an adjustable lumen size and/or restriction designed to adjust the flowrate of blood through the clamp 26. The clamp 26 may be designed such that a first section of tubing (e.g., flexible, semi-rigid, rigid tubing) may be inserted into an inlet of the clamp 26 and a second section of tubing may be inserted into an outlet of the clamp 26. Accordingly, the blood may flow through the first section of tubing into the clamp 26, through a valve located in the clamp 26, and exit the clamp 26 via an outlet of the clamp 26 and into the second section of tubing.

FIG. 1 further illustrates that the extracorporeal perfusion system 10 may include a flow sensor 22 positioned along the venous blood pathway 34. In some examples the flow sensor 22 may be fixedly attached to the clamp 26 (e.g., the flow sensor 22 may be an integrated component of the clamp 26). However, in other examples the flow sensor 22 may be a separate and distinct component, separated from the clamp 26 and positioned along any portion of the venous blood pathway 34. In some examples, the flow sensor 22 may be positioned on an inner surface, the outer surface or within a wall of the tubing defining the venous blood pathway 34. In other examples, the flow sensor 22 may be positioned adjacent to a component (e.g., tubing) defining the blood pathway 34.

The flow sensor 22 may be a flow sensor configured to sense (e.g., detect, measure, compute, monitor, etc.) the flowrate of blood passing through the blood pathway 34. Additionally, the extracorporeal perfusion system 10 may include additional sensors positioned along the blood pathway 34. For example, the extracorporeal perfusion system 10 may include one or more sensors for monitoring pressures, temperatures, bubbles, oxygen saturation, carbon dioxide content, blood gases, or other blood parameters. Further, in some instances a single sensor may be configured to sense multiple blood parameters including blood flowrate, pressures, temperatures, bubbles, oxygen saturation, carbon dioxide content, blood gases, etc.

FIG. 1 further illustrates that the extracorporeal perfusion system 10 may include a pressure sensor 24 positioned along the venous blood pathway 34 and/or the blood reservoir 18. In some examples the pressure sensor 24 may be fixedly attached to the clamp 26 (e.g., the pressure sensor 24 may be an integrated component of the clamp 26). However, in other examples the pressure sensor 24 may be a separate and distinct component, separated from the clamp 26 and positioned along any portion of the venous blood pathway 34. In some examples, the pressure sensor 24 may be positioned on an inner surface, the outer surface or within a wall of the tubing defining the venous blood pathway 34. In other examples, the pressure sensor 24 may be positioned adjacent to a component (e.g., tubing) defining the blood pathway 34. The pressure sensor 24 may be a pressure sensor configured to sense (e.g., detect, measure, compute, monitor, etc.) the pressure of blood passing through the blood pathway 34.

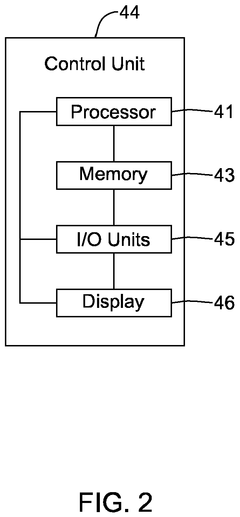

As discussed herein, the extracorporeal perfusion system 10 may also include a control unit 44. The control unit 44 may include a visual display 46 and/or one or more control knob (e.g., buttons). FIG. 2 further illustrates that the control unit 44 may include, among other suitable components, a processor 41, memory 43, and an I/O unit 45.

The processor 41 of the control unit 44 may include a single processor or more than one processor working individually or with one another. The processor 41 may be configured to execute instructions, including instructions that may be loaded into the memory 43 and/or other suitable memory. Example processor components may include, but are not limited to, microprocessors, microcontrollers, multi-core processors, graphical processing units, digital signal processors, application specific integrated circuits (ASICs), field programmable gate arrays (FPGAs), discrete circuitry, and/or other suitable types of data processing devices.

The memory 43 of the control unit 44 may include a single memory component or more than one memory component each working individually or with one another. Example types of memory may include random access memory (RAM), EEPROM, FLASH, suitable volatile storage devices, suitable non-volatile storage devices, persistent memory (e.g., read only memory (ROM), hard drive, Flash memory, optical disc memory, and/or other suitable persistent memory) and/or other suitable types of memory. The memory 43 may be or may include a non-transitory computer readable medium.

The I/O units 45 of the control unit 44 may include a single I/O component or more than one I/O component each working individually or with one another. Example I/O units 45 may be any type of communication port configured to communicate with other components of the building management system. Example types of I/O units 45 may include wired ports, wireless ports, radio frequency (RF) ports, Low-Energy Bluetooth ports, Bluetooth ports, Near-Field Communication (NFC) ports, HDMI ports, Wi-Fi ports, Ethernet ports, VGA ports, serial ports, parallel ports, component video ports, S-video ports, composite audio/video ports, DVI ports, USB ports, optical ports, and/or other suitable ports.

Additionally, the control unit 44 may be in communication with various components of the extracorporeal perfusion system 10. For example, FIG. 1 illustrates that the control unit 44 may be in communication (e.g., wireless, wired communication, or other communication means capable of transmitting signals) with the clamp 26, the flow sensor 22 and the pressure sensor 24, all of which may be positioned along the venous blood pathway 34. FIG. 1 depicts the flow sensor 22 in communication with the control unit 44 via a dashed line 30. Further, FIG. 1 depicts the pressure sensor 24 in communication with the control unit 44 via a dashed line 32. In some examples, the control unit 44 may be integrated into a console or work station of the extracorporeal perfusion system 10. In other examples, the control unit 44 may be integrated directly into a heart-lung machine. The control unit 44 may be in direct or indirect communication with a console, work station and/or a heart-lung machine.

Further, the clamp 26, the flow sensor 24 and/or the pressure sensor 22 and the control unit 44 may together form a closed-loop system capable of automatically or manually regulating the flowrate of blood within the venous blood pathway 34. In some examples, the flowrate within the blood pathway may be from 0-8 liters/min at a pressure between -200 mmHg and +800 mmHg. For example, the flow sensor 22 and/or the pressure sensor 24 may be configured to sense a first parameter (e.g., flowrate, pressure etc.) of blood passing through the blood pathway 34. Additionally, the flow sensor 22 and/or the pressure sensor 24 may be configured to transmit a signal corresponding to the sensed parameter (e.g., flowrate, pressure, etc.) to the control unit 44. Further, the control unit 44 may be configured to receive the signal (corresponding to the sensed flowrate and/or pressure of the blood within the blood pathway 34) transmitted by the flow sensor 22 and/or the pressure sensor 24. The control unit 44 may be configured to compare the signal received from the flow sensor 22 and/or the pressure sensor 24 to a parameter (e.g., flowrate, pressure, etc.) set point input by a clinician into the control unit 44. After comparing the signal received from the flow sensor 22 and/or the pressure sensor 24, the control unit 44 may transmit a signal to the clamp 26. The clamp 26 may be configured to receive the signal from the control unit 44. After receiving and processing the signal from the control unit 44, the clamp 26 may be automatically actuated to adjust the blood flow (e.g., the flowrate of the blood) through the blood pathway 34 in response to receiving the signal from the control unit 44. In some examples, the clamp 26 may send a signal back to the control unit 44 confirming the position to which the aperture of the clamp 26 has been actuated (e.g., the clamp 26 may send a signal indicating the size of the aperture through which the blood is flowing, such as a percentage that that the clamp 26 is opened). It can be appreciated that a component (e.g., console unit 44, clamp 26, flow sensor 22, pressure sensor 24, etc.) of the extracorporeal perfusion system 10 may include an algorithm which utilizes the sensed flowrate data from the flow sensor 22 and/or sensed pressure data from the pressure sensor 24 to calculate the appropriate automatic actuation of the clamp 26 required to meet the clinician's desired blood flowrate within the blood pathway 34. It can be further appreciated that the set point and/or set range of values for the flowrate of blood through the venous blood pathway 34 may be input by a clinician via the control features (e.g., display, dial, button, etc.) 46 of the control unit 44 or other components of the extracorporeal perfusion system 10 (e.g., heart-lung machine). In other words, a clinician may be able to input a set point or a set range of values for blood flowrates in various blood pathways in the system via a touchpad, dial, control knob, etc.

As discussed herein, after receiving and processing the signal from the control unit 44, the clamp 26 may be automatically actuated to adjust the blood flowrate through the blood pathway 34 in response to receiving the signal from the control unit 44. In some examples, a component (e.g., console unit 44, clamp 26, flow sensor 22, pressure sensor 24, etc.) of the extracorporeal perfusion system 10 may include an algorithm which utilizes sensed blood pressure data from the pressure sensor 24 to calculate the appropriate automatic actuation of the clamp 26 required to meet the clinician's desired blood pressure within the blood pathway 34. It can be appreciated that the set point or set range of values for the pressure of blood through the venous blood pathway 34 may be input by a clinician via the display 46 of the control unit 44. In other words, a clinician may be able to input a set point or set range of values for blood pressure in various blood pathways in the system via a touchpad, dial, control knob, etc.

As described herein, the control unit 44 (and all control units described herein) may permit a user (e.g., perfusionist, clinician, etc.) to input pre-defined values or a pre-defined range of values for the flowrate of blood within the venous blood pathway 34 (independent of the arterial blood pathway 42), the arterial blood pathway 42 (independent of the venous blood pathway 34) or both the venous blood pathway 34 and the arterial blood pathway 42. In some examples, the flowrate of blood in the venous blood pathway 34 may be regulated by the clamp 26 positioned in the venous blood pathway 34. The actuation of the clamp 26 may control the flowrate of blood in the venous blood pathway 34, whereby the actuation of the clamp 26 is determined by a flowrate of venous blood as measured by the flow sensor 22. Additionally, the actuation of the clamp 26 may control the flowrate of blood in the venous blood pathway 34, whereby the actuation of the clamp 26 is determined by a pressure of the venous blood as measured by the pressure sensor 24 (e.g., the pressure of the blood in the venous blood pathway 34 may be correlated to the flowrate of blood within the venous blood pathway 34).

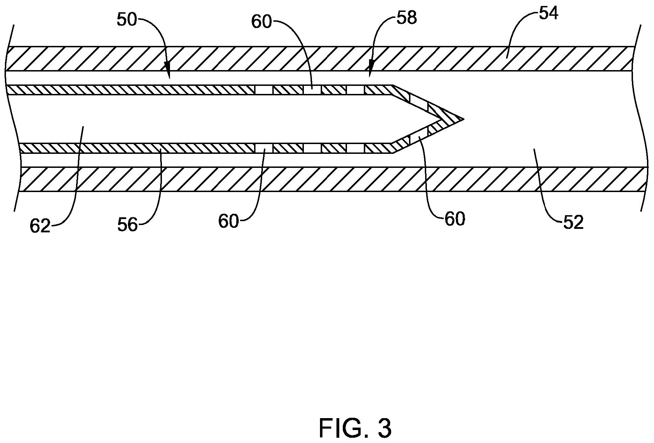

As set forth above, it can be appreciated that removing the blood from the patient may include placing multiple cannulae into the patient's body in a variety of locations, depending on the type of medical procedure being performed. For example, FIG. 3 illustrates an example cannula 50 placed within a body vessel 54 (e.g., the superior vena cava and/or inferior vena cava) of a patient, wherein the cannula is configured to remove deoxygenated blood from the patient's body. FIG. 3 further illustrates that the cannula 50 may include a body portion 56 and a distal end region 58. In some examples, the distal end region 58 may include a tapered portion. For example, FIG. 3 illustrates the distal end region 58 having a generally conical shape whereby the distal end region tapers from a first diameter to a tapered point. FIG. 3 further illustrates that the cannula 50 may include one or more apertures 60 extending through the wall of the cannula 50. FIG. 3 illustrates that, in some examples, the cannula may include one or more apertures 60 positioned on the body portion 56 of the cannula 50 and/or one or more apertures 60 positioned on the distal end region 58.

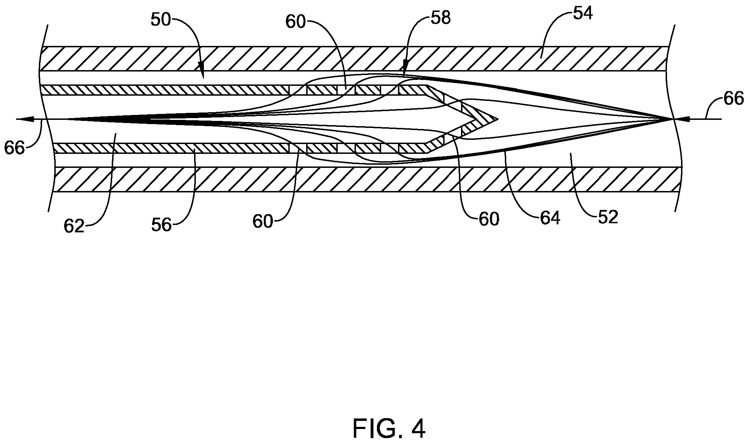

As illustrated in FIG. 4, it can be appreciated that the apertures 60 may permit blood 52 to flow from a position outside the cannula 50, through the wall of the cannula 50 and into the lumen 62 of the cannula 50. For example, FIG. 4 depicts the flow of blood via the lines 64, whereby the blood is flowing in the direction indicated by the arrows 66. As discussed herein, FIG. 4 illustrates the blood flowing from a position outside the cannula 50, through the wall of the cannula 50 and into the lumen 62 of the cannula 50. The blood may travel through the lumen 62 of the cannula 50 along the blood pathway 34 (shown in FIG. 1) and into the reservoir 18.

It can further be appreciated that in order for blood to be drained from the patient, a negative pressure has to be created at the distal end region 58 of the cannula 50. It can be appreciated that in FIG. 4, the pressure outside the distal end region 58 of the cannula 50 may be greater than the pressure in the lumen 62 of the cannula, whereby the blood 52 may flow from the area of higher pressure outside the cannula 50 to the area of lower pressure inside the lumen 62 of the cannula 50. As will be discussed herein, the negative pressure may be created in a variety of ways, including via gravitation, a kinetic assist device (e.g., pump) and a vacuum module. However, as discussed herein, in some instances the suction created by the negative pressure gradient may force the vessel 54 to collapse onto the outer surface of the cannula 50, thereby blocking the apertures 60 and effectively blocking the flow of blood within the blood pathway 34.

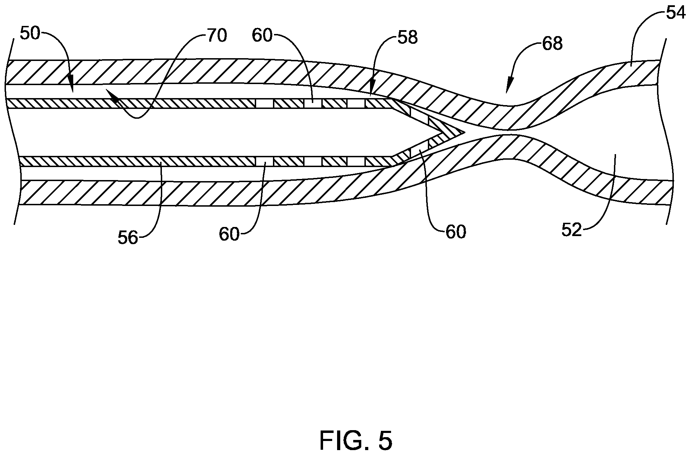

For example, FIG. 5 illustrates suction created by the negative pressure gradient forcing the vessel 54 to collapse onto the outer surface of the cannula 50, thereby blocking the apertures 60 and effectively blocking the flow of blood within the blood pathway 34. FIG. 5 illustrates that the vessel 54 may collapse onto the outer surface of the cannula 50, whereby the inner surface 70 of the vessel 54 may contact the outer surface of the cannula 50 and block the apertures 60. Additionally, FIG. 5 illustrates that the vessel 54 may collapse to an extent such that a blockage 68 may occur at a position distal to the distal end region 58 of the cannula 50. The blockage may effectively stop the flow of blood 52 with the blood vessel 54. It can be appreciated that the negative pressure gradient (e.g., the negative pressure within the lumen 62 of the cannula 50 relative to the pressure outside the cannula 50) may keep the cannula in place and prevent the wall of the blood vessel 54 from releasing from the outside surface of the cannula 50. It can be further appreciated that without intervention, the blood may collect distal to the blockage 68 and the vessel 54 may eventually detach from the outer surface of the cannula 50. However, as long as the negative pressure gradient remains between the lumen 62 of the cannula 50 and the region distal to the distal end region 58 of the cannula 50, the vessel 54 will continue to collapse onto the outer surface of the cannula 50 and the blockage 68 will repeatedly occur. Therefore, it may be desirable to design an extracorporeal perfusion system which may include one or more cannula blockage detection systems configured to monitor the flow of blood within the extracorporeal perfusion system and automatically unblock a blocked cannula.

It can be appreciated that getting the vessel 54 to release from the outer surface of the cannula 50 (and thereby regain blood flow within the vessel 54) may require utilizing one or more elements to reduce the negative pressure gradient located at the distal end region 58 of the cannula 50. Referring to FIG. 1, the negative pressure gradient located at the distal end region 58 of the cannula 50 may be reduced by slowly closing the clamp 26. It can be appreciated that slowly closing the clamp 26 may reduce the blood flow within the lumen 62 of the cannula 50, thereby increasing the pressure within the lumen 62 of the cannula 50 at the distal end region 58. It can be appreciated that increasing the pressure within the lumen 62 of the cannula 50 at the distal end region 58 may reduce the negative pressure gradient at the distal end region 58 of the cannula 50, thereby creating an equilibrium of pressure between the blood positioned within the lumen 62 of the cannula 50 and the pressure of the blood positioned within the vessel 54 at a location distal to the distal end region 58 of the cannula 50. Equalizing the pressure between the blood positioned within the lumen 62 of the cannula 50 and the pressure of the blood positioned within the vessel 54 at a location distal to the distal end region 58 of the cannula 50 may permit the vessel 54 to release from the cannula 50 and permit blood to resume flow from the vessel 54, through the apertures 60 and into the lumen 62 of the cannula 50 (as illustrated in FIG. 4).

Additionally, detection of a cannula blockage may be accomplished by monitoring the blood flow within the venous blood pathway 34. For example, the blood flow sensor 22 may be utilized to automatically sense the blood flow within the venous blood pathway and send the sensed blood flow data to the control unit 44. In turn, the control unit 44 may receive the sensed blood flow within the blood pathway 34 and sense a drop (or a series of repeated drops in blood flow) in the blood flow within the blood pathway 34. For example, the control unit 44 may include one or more processing algorithms designed to sense a drop (or a series of repeated drops in blood flow) in the blood flow within the blood pathway 34. Upon sensing a drop in the blood flow within the blood pathway 34, the control unit 44 may also output a visual indication of the sensed drop in blood flow to the display 46, thereby communicating the sensed drop in the blood flow within the blood pathway 34 to a clinician observing the system. Further, upon sensing a drop (or a series of repeated drops in blood flow) in the blood flow within the blood pathway 34, the control unit 44 may send a signal to the clamp 26, whereby the clamp 26 may gradually close in response to receiving the signal from the control unit 44. As discussed herein, gradually closing the clamp 26 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50. Further, upon automatic closure of the clamp 26, the control unit 44 may also output a visual indication of the closing of the clamp 26 to the display 46, thereby communicating the closing of the clamp 26 and resumption of blood flow to a clinician observing the system. Further yet, in some examples, the control unit 44 may also send a communication to the display 46 indicating that blood flow in the arterial blood flow should also be decreased.

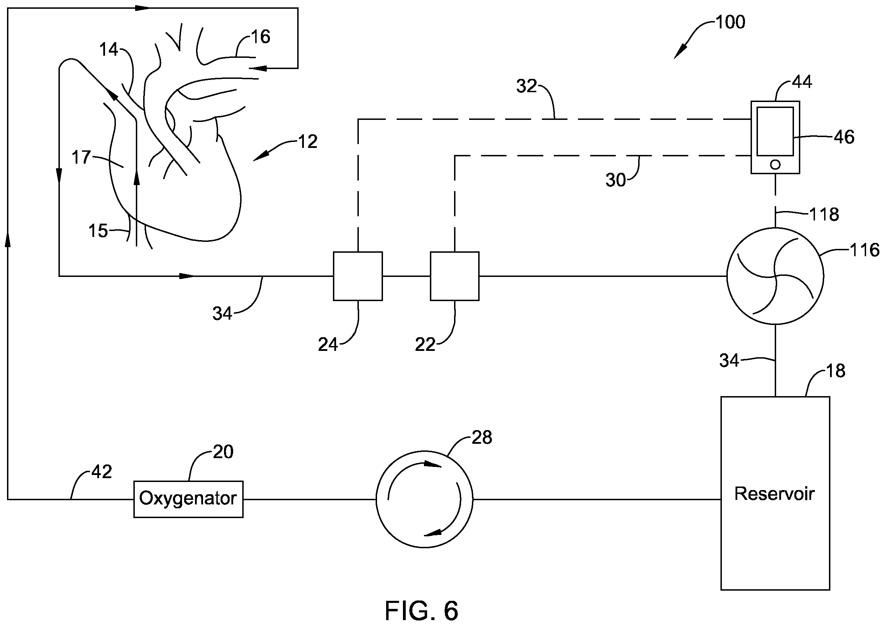

Similarly, detection of a cannula blockage may be accomplished by monitoring the blood pressure within the venous blood pathway 34. For example, the blood pressure sensor 24 may be utilized to automatically sense the blood pressure within the venous blood pathway and send the sensed blood pressure data to the control unit 44. In turn, the control unit 44 may receive the sensed blood pressure within the blood pathway 34 and sense a drop (or a series of repeated drops) in the blood pressure within the blood pathway 34. For example, the control unit 44 may include one or more processing algorithms designed to sense a drop (or a series of repeated drops) in the blood pressure within the blood pathway 34. Upon sensing a drop in the blood pressure within the blood pathway 34, the control unit 44 may also output a visual indication of the sensed drop in blood pressure to the display 46, thereby communicating the sensed drop in the blood pressure within the blood pathway 34 to a clinician observing the system. Further, upon sensing a drop (or a series of repeated drops) in the blood pressure within the blood pathway 34, the control unit 44 may send a signal to the clamp 26, whereby the clamp 26 may gradually close in response to receiving the signal from the control unit 44. As discussed herein, gradually closing the clamp 26 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50. Further, upon automatic closure of the clamp 26, the control unit 44 may also output a visual indication of the closing of the clamp 26 to the display 46, thereby communicating the closing of the clamp 26 and resumption of blood pressure to a clinician observing the system. Further yet, in some examples, the control unit 44 may also send a communication to the display 46 indicating that blood pressure in the arterial blood flow should also be decreased. FIG. 6 illustrates another example extracorporeal perfusion system 100. Similar to other perfusion systems described herein, the components of the extracorporeal perfusion system 100 described herein may be interconnected in a variety of configurations to monitor and regulate blood flow through the various blood pathways of the extracorporeal perfusion system 100.

For example, FIG. 6 illustrates the extracorporeal perfusion system 100 in which the control unit 44 is in communication (e.g., wired, wireless communication, or other communication means capable of transmitting signals) with the flow sensor 22 (positioned along the venous return pathway 34), the pressure sensor 24 (positioned along the venous return pathway 34), and a pump 116 (e.g., a centrifugal pump, a roller pump, etc.). Similar to other systems described herein, the flow sensor 22, the pressure sensor 24, the pump 116 and the control unit 44 may together form a first closed-loop system capable of regulating the flowrate of blood within the venous blood pathway 34. Similar to that discussed herein with respect to FIG. 1, the gravitational blood flow from a patient to the reservoir 18 may be insufficient to support adequate blood flow through the extracorporeal perfusion circuit. Accordingly, in some instances the pump 116 may be utilized to increase blood flow to the reservoir 18. The pumping action of the pump 116 may be manually or automatically increased or decreased to adjust the blood flowrate through the venous blood pathway 34 in response to receiving the signal from the control unit 44.

Similarly to the extracorporeal perfusion system 10 described above with respect to FIG. 1, the extracorporeal perfusion system 100 may utilize one or more elements to reduce a negative pressure gradient located at the distal end region 58 of the cannula 50. Referring to FIG. 6, the negative pressure gradient located at the distal end region 58 of the cannula 50 may be reduced by slowly reducing the speed of the pump 116. It can be appreciated that slowly reducing the speed of the pump 116 may reduce the blood flow within the lumen 62 of the cannula 50, thereby increasing the pressure within the lumen 62 of the cannula 50 at the distal end region 58. It can be appreciated that increasing the pressure within the lumen 62 of the cannula 50 at the distal end region 58 may reduce the negative pressure gradient at the distal end region 58 of the cannula 50, thereby creating an equilibrium of pressure between the blood positioned within the lumen 62 of the cannula 50 and the pressure of the blood positioned within the vessel 54 at a location distal to the distal end region 58 of the cannula 50. Equalizing the pressure between the blood positioned within the lumen 62 of the cannula 50 and the pressure of the blood positioned within the vessel 54 at a location distal to the distal end region 58 of the cannula 50 may permit the vessel 54 to release from the cannula 50 and permit blood to resume flow from the vessel 54, through the apertures 60 and into the lumen 62 of the cannula 50 (as illustrated in FIG. 4).

Additionally, detection of a cannula blockage may be accomplished by monitoring the blood flow and/or the blood pressure within the venous blood pathway 34. For example, the blood flow sensor 22 may be utilized to automatically sense the blood flow within the venous blood pathway and send the sensed blood flow data to the control unit 44. In turn, the control unit 44 may receive the sensed blood flow within the blood pathway 34 and sense a drop (or a series of repeated drops in blood flow) in the blood flow within the blood pathway 34. For example, the control unit 44 may include one or more processing algorithms designed to sense a drop (or a series of repeated drops in blood flow) in the blood flow within the blood pathway 34. Upon sensing a drop in the blood flow within the blood pathway 34, the control unit 44 may also output a visual indication of the sensed drop in blood flow to the display 46, thereby communicating the sensed drop in the blood flow within the blood pathway 34 to a clinician observing the system. Further, upon sensing a drop (or a series of repeated drops in blood flow) in the blood flow within the blood pathway 34, the control unit 44 may send a signal to the pump 116, whereby the speed of the pump 116 may gradually decrease in response to receiving the signal from the control unit 44. As discussed herein, gradually reducing the speed of the pump 116 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50. Further, upon automatically reducing the speed of the pump 116, the control unit 44 may also output a visual indication of the reduction in the speed of the pump 116 to the display 46, thereby communicating the reduction of the speed of the pump 116 and resumption of blood flow to a clinician observing the system. Further yet, in some examples, the control unit 44 may also send a communication to the display 46 indicating that blood flow in the arterial blood flow should also be decreased.

Similarly, detection of a cannula blockage may be accomplished by monitoring the blood pressure within the venous blood pathway 34 and/or at the blood reservoir 18. For example, the blood pressure sensor 24 may be utilized to automatically sense the blood pressure within the venous blood pathway and send the sensed blood pressure data to the control unit 44. In turn, the control unit 44 may receive the sensed blood pressure within the blood pathway 34 and sense a drop (or a series of repeated drops in blood pressure) in the blood pressure within the blood pathway 34. For example, the control unit 44 may include one or more processing algorithms designed to sense a drop (or a series of repeated drops in blood pressure) in the blood pressure within the blood pathway 34. Upon sensing a drop in the blood pressure within the blood pathway 34, the control unit 44 may also output a visual indication of the sensed drop in blood pressure to the display 46, thereby communicating the sensed drop in the blood pressure within the blood pathway 34 to a clinician observing the system. Further, upon sensing a drop (or a series of repeated drops in blood pressure) in the blood pressure within the blood pathway 34, the control unit 44 may send a signal to the pump 116, whereby the speed of the pump 116 may gradually decrease in response to receiving the signal from the control unit 44. As discussed herein, gradually reducing the speed of the pump 116 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50. Further, upon automatically reducing the speed of the pump 116, the control unit 44 may also output a visual indication of the reduction in the speed of the pump 116 to the display 46, thereby communicating the reduction of the speed of the pump 116 and resumption of blood pressure to a clinician observing the system. Further yet, in some examples, the control unit 44 may also send a communication to the display 46 indicating that blood pressure in the arterial blood flow should also be decreased.

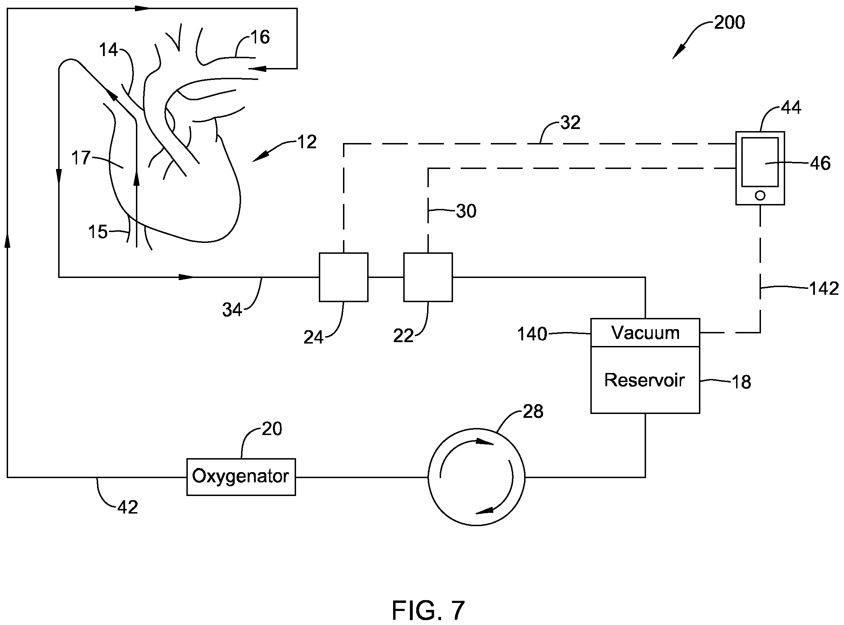

FIG. 7 illustrates another example extracorporeal perfusion system 200. Similar to other perfusion systems described herein, the components of the extracorporeal perfusion system 200 described herein may be interconnected in a variety of configurations to monitor and regulate blood flow through the various blood pathways of the extracorporeal perfusion system 200.

For example, FIG. 7 illustrates the extracorporeal perfusion system 200 in which the control unit 44 is in communication (e.g., wired, wireless communication, or other communication means capable of transmitting signals) with the flow sensor 22 (e.g., positioned along the venous return pathway 34), the pressure sensor 24 (e.g., positioned along the venous return pathway 34 and/or the blood reservoir 18) and a vacuum unit 140 (e.g., positioned along the venous return pathway 34). In some examples, the vacuum unit 140 may be directly coupled to the reservoir 18. However, in other examples, the vacuum unit 140 may be spaced away from the reservoir 18. As illustrated by the dashed line 142, the vacuum unit 140 may be in communication (e.g., wired, wireless communication, or other communication means capable of transmitting signals) with the control unit 44. It can be appreciated that the vacuum unit 140 may assist in the flow of blood from the patient to the reservoir 18. For example, the vacuum unit 140 may be utilized to create a negative pressure that effectively pulls blood from the patient and into the blood reservoir 18. In some examples, the vacuum unit 140 may be utilized in conjunction with a gravitational flow system, while in other examples, the vacuum unit 140 may be utilized without a gravitational flow system. Similar to other systems described herein, the flow sensor 22, the pressure sensor 24, the vacuum unit 140 and the control unit 44 may together form a first closed-loop system capable of regulating the flowrate of blood within the venous blood pathway 34.

In some examples, the control unit 44 of the extracorporeal perfusion system 800 may be designed to receive signals from the flow sensor 22 and/or the pressure sensor 24 and adjust the vacuum unit 140 based on the signals received from the flow sensor 22 and/or the pressure sensor 24. For example, a user may set the control unit 44 to maintain a desired flowrate within the venous blood pathway 34. In response, the control unit 44 may monitor pressure signals received from the flow sensor 22 and/or the pressure sensor 24 and adjust the negative pressure generated by the vacuum unit 140 to maintain the blood flowrate with the venous blood pathway 34 at the desired level.

Similarly to that described above with respect to FIG. 1 and FIG. 6, the extracorporeal perfusion system 200 of FIG. 7 may utilize one or more elements to reduce a negative pressure gradient located at the distal end region 58 of the cannula 50. Referring to FIG. 7, the negative pressure gradient located at the distal end region 58 of the cannula 50 may be reduced by slowly reducing the suction of the vacuum unit 140. It can be appreciated that slowly reducing the suction of the vacuum unit 140 may reduce the blood flow within the lumen 62 of the cannula 50, thereby increasing the pressure within the lumen 62 of the cannula 50 at the distal end region 58. It can be appreciated that increasing the pressure within the lumen 62 of the cannula 50 at the distal end region 58 may reduce the negative pressure gradient at the distal end region 58 of the cannula 50, thereby creating an equilibrium of pressure between the blood positioned within the lumen 62 of the cannula 50 and the pressure of the blood positioned within the vessel 54 at a location distal to the distal end region 58 of the cannula 50. Equalizing the pressure between the blood positioned within the lumen 62 of the cannula 50 and the pressure of the blood positioned within the vessel 54 at a location distal to the distal end region 58 of the cannula 50 may permit the vessel 54 to release from the cannula 50 and permit blood to resume flow from the vessel 54, through the apertures 60 and into the lumen 62 of the cannula 50 (as illustrated in FIG. 4).

Additionally, detection of a cannula blockage may be accomplished by monitoring the blood flow and/or the blood pressure within the venous blood pathway 34. For example, the blood flow sensor 22 may be utilized to automatically sense the blood flow within the venous blood pathway and send the sensed blood flow data to the control unit 44. In turn, the control unit 44 may receive the sensed blood flow within the blood pathway 34 and sense a drop (or a series of repeated drops in blood flow) in the blood flow within the blood pathway 34. For example, the control unit 44 may include one or more processing algorithms designed to sense a drop (or a series of repeated drops in blood flow) in the blood flow within the blood pathway 34. Upon sensing a drop in the blood flow within the blood pathway 34, the control unit 44 may also output a visual indication of the sensed drop in blood flow to the display 46, thereby communicating the sensed drop in the blood flow within the blood pathway 34 to a clinician observing the system. Further, upon sensing a drop (or a series of repeated drops in blood flow) in the blood flow within the blood pathway 34, the control unit 44 may send a signal to the vacuum unit 140, whereby the suction of the vacuum unit 140 may gradually decrease in response to receiving the signal from the control unit 44. As discussed herein, gradually reducing the suction of the vacuum unit 140 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50. Further, upon automatically reducing the suction of the vacuum unit 140, the control unit 44 may also output a visual indication of the reduction in the suction of the vacuum unit 140 to the display 46, thereby communicating the reduction of the suction of the vacuum unit 140 and resumption of blood flow to a clinician observing the system. Further yet, in some examples, the control unit 44 may also send a communication to the display 46 indicating that blood flow in the arterial blood flow should also be decreased.

Similarly, detection of a cannula blockage may be accomplished by monitoring the blood pressure within the venous blood pathway 34. For example, the blood pressure sensor 24 may be utilized to automatically sense the blood pressure within the venous blood pathway and send the sensed blood pressure data to the control unit 44. In turn, the control unit 44 may receive the sensed blood pressure within the blood pathway 34 and sense a drop (or a series of repeated drops in blood pressure) in the blood pressure within the blood pathway 34. For example, the control unit 44 may include one or more processing algorithms designed to sense a drop (or a series of repeated drops in blood pressure) in the blood pressure within the blood pathway 34. Upon sensing a drop in the blood pressure within the blood pathway 34, the control unit 44 may also output a visual indication of the sensed drop in blood pressure to the display 46, thereby communicating the sensed drop in the blood pressure within the blood pathway 34 to a clinician observing the system. Further, upon sensing a drop (or a series of repeated drops in blood pressure) in the blood pressure within the blood pathway 34, the control unit 44 may send a signal to the pump 116, whereby the suction of the vacuum unit 140 may gradually decrease in response to receiving the signal from the control unit 44. As discussed herein, gradually reducing the suction of the vacuum unit 140 may create an equilibrium of pressure between the blood positioned within the lumen 62 of the cannula 50 and the pressure of the blood positioned within the vessel 54 at a location distal to the distal end region 58 of the cannula 50, and thereby resume blood flow from the vessel 54, through the apertures 60 and into the lumen 62 of the cannula 50

It can further be appreciated that automatically sensing a drop in blood flow and/or blood pressure and reducing the negative pressure via gradually closing the clamp 26 in the system 10, gradually reducing the speed of the pump 140 in system 100, or gradually reducing the suction of the vacuum unit in the system 200, may reduce the downtime of the blood flow (e.g., the blood flow with the venous pathway 34 remains consistent as compared to the stopping and restarting of blood flow when the cannula is repeatedly blocked) may enable a perfusionist to remain focused on the medical procedure rather than having to manually intervene and remedy repeated blockages and the resulting reduction in blood flow. Additionally, preventing the stoppage of blood flow within the blood pathway 34 may prevent undesirable damage to the red blood cells of the patient. Further, upon automatically closing the clamp 26 in the system 10, gradually reducing the speed of the pump 140 in system 100, or gradually reducing the suction of the vacuum unit in the system 200, the control unit 44 may also output a visual indication of the closing the clamp 26 in the system 10, gradually reducing the speed of the pump 140 in system 100, or gradually reducing the suction of the vacuum unit in the system 200 to the display 46, thereby communicating the closing the clamp 26 in the system 10, gradually reducing the speed of the pump 140 in system 100, or gradually reducing the suction of the vacuum unit in the system 200and resumption of blood pressure to a clinician observing the system.

Additionally, it can be appreciated that one or more actions including the closing of the clamp 26 in the system 10, gradually reducing the speed of the pump 140 in system 100, or gradually reducing the suction of the vacuum unit in the system 200 may be performed in isolation or in any combination with each other to maintain the blood flowrate with the venous blood pathway 34 at the desired level. For example, the system may close the clamp 26 in the system 10 in combination with gradually reducing the speed of the pump 140 in system 100, or the system may close the clamp 26 in the system 10 in combination with gradually reducing the suction of the vacuum unit in the system 200, or the system may gradually reduce the speed of the pump 140 in system 100 in combination with gradually reducing the suction of the vacuum unit in the system 200. In yet other examples, the system may close of the clamp 26 in the system 10, gradually reducing the speed of the pump 140 in system 100 and also gradually reduce the suction of the vacuum unit in the system 200 to maintain the blood flowrate with the venous blood pathway 34 at the desired level.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is defined in the language in which the appended claims are expressed.

## Claims

1. An extracorporeal blood treatment system, comprising:
a cannula positioned within a venous blood pathway extending between the patient and a reservoir, wherein the cannula includes a distal end region and a lumen extending therein;
a first sensor positioned along the venous blood pathway;
a control unit in communication with the first sensor;
wherein the first sensor is configured to sense a first sensor parameter of blood passing through the venous blood pathway;
wherein the first sensor is configured to automatically transmit a first signal corresponding to the first sensor parameter to the control unit;
wherein the control unit is configured to receive the first signal and automatically transmit a second signal to a first control element;
wherein the first control element is configured to receive the second signal from the control unit;
wherein the first control element is configured to reduce the blood flow within the venous blood pathway in response to receiving the second signal, and wherein reducing the blood flow substantially equalizes a blood pressure within the lumen of the distal end region of the cannula and the blood pressure of a region of the venous blood pathway adjacent to the distal end region of the cannula.

2. The blood treatment system of claim 1, wherein the first sensor is a blood flow sensor.

3. The blood treatment system of claim 2, wherein the first sensor parameter is a flowrate of blood passing through the venous blood pathway.

4. The blood treatment system of claim 1, wherein the first sensor is a blood pressure sensor.

5. The blood treatment system of claim 1, wherein the first sensor parameter is a pressure of blood passing through the venous blood pathway, the reservoir or both the venous blood pathway and the reservoir.

6. The blood treatment system of claim 1, wherein the first control element includes a clamp, and wherein the clamp is configured to decrease the flowrate of blood flowing through the venous blood pathway in response to receiving the second signal from the control unit.

7. The blood treatment system of claim 6, wherein the control unit is configured to automatically actuate the clamp in response to receiving the first signal from the first sensor.

8. The blood treatment system of claim 1, wherein the first control element includes a pump, and wherein the pump is configured to adjust the flowrate of blood flowing through the venous blood pathway in response to receiving the second signal from the control unit.

9. The blood treatment system of claim 8, wherein the control unit is configured to automatically decrease a speed of the pump in response to receiving the first signal from the first sensor.

10. The blood treatment system of claim 1, wherein the first control element includes a vacuum unit, and wherein the vacuum unit is configured to adjust the flowrate of blood flowing through the venous blood pathway in response to receiving the second signal from the control unit.

11. The blood treatment system of claim 10, wherein the control unit is configured to automatically reduce the suction of the vacuum unit in response to receiving the first signal from the first sensor.

12. The blood treatment system of claim 1, wherein equalizing the blood pressure within the lumen of the distal end region of the cannula and the blood pressure of a region of the venous blood pathway adjacent to the distal end region of the cannula restores blood flow within the lumen of the cannula.

13. An extracorporeal blood treatment system, comprising:
a cannula positioned within a venous blood pathway extending between the patient and a reservoir, wherein the cannula includes a distal end region and a lumen extending therein;
a first sensor positioned along the venous blood pathway;
a second sensor positioned along the venous blood pathway;
a control unit in communication with the first sensor and the second sensor;
wherein the first sensor is configured to sense a first sensor parameter of blood passing through the venous blood pathway;
wherein the second sensor is configured to sense a second sensor parameter of blood passing through the venous blood pathway;
wherein the first sensor is configured to transmit a first signal corresponding to the first sensor parameter to the control unit;
wherein the second sensor is configured to transmit a second signal corresponding to the second sensor parameter to the control unit;
wherein the control unit is configured to receive the first signal and transmit a third signal to a first control element;
wherein the control unit is configured to receive the second signal and transmit a fourth signal to the first control element;
wherein the first control element is configured to receive the third signal and the fourth signal from the control unit;
wherein the first control element is configured to reduce the blood flow within the venous blood pathway in response to receiving the third signal, the fourth signal or both the third and the fourth signals, and wherein reducing the blood flow substantially equalizes a blood pressure within the lumen of the distal end region of the cannula and the blood pressure of a region of the venous blood pathway adjacent to the distal end region of the cannula.

14. An extracorporeal blood treatment system, comprising:
a first control element coupled to a venous blood pathway extending between a patient and a reservoir;
a blood flowrate sensor positioned along the venous blood pathway;
a blood pressure sensor positioned along the venous blood pathway; and
a control unit in communication with the blood flowrate sensor, the blood pressure sensor, and the first control element;
wherein the blood flowrate sensor is configured to automatically transmit a first signal to the control unit, wherein the first signal corresponds to a flowrate of blood in the venous blood pathway;
wherein the blood pressure sensor is configured to automatically transmit a second signal to the control unit, wherein the second signal corresponds to a pressure of blood in the venous blood pathway;
wherein the control unit is configured to automatically receive the first signal and the second signal;
wherein the control unit is configured to automatically send a third signal to the first control element in response to receiving the first signal, the second signal or both the first and the second signals;
wherein the first control element is configured to automatically reduce the blood flow within the venous blood pathway in response to receiving the third signal, and wherein reducing the blood flow substantially equalizes a blood pressure within the lumen of the distal end region of the cannula and the blood pressure of a region of the venous blood pathway adjacent to the distal end region of the cannula.

15. An extracorporeal blood treatment system, comprising:
a cannula positioned within a venous blood pathway extending between the patient and a reservoir, wherein the cannula includes a distal end region and a lumen extending therein;
a first sensor positioned along the venous blood pathway;
a control unit in communication with the first sensor;
wherein the first sensor is configured to sense a first sensor parameter of blood passing through the venous blood pathway;
wherein the first sensor is configured to automatically transmit a first signal corresponding to the first sensor parameter to the control unit;
wherein the control unit is configured to receive the first signal and automatically transmit a second signal to one or more of a clamp, a pump and a vacuum unit;
wherein one or more of the clamp, the pump and the vacuum unit are configured to receive the second signal from the control unit;
wherein one or more of the clamp, the pump and the vacuum unit are configured to reduce the blood flow within the venous blood pathway in response to receiving the second signal, and wherein reducing the blood flow substantially equalizes a blood pressure within the lumen of the distal end region of the cannula and the blood pressure of a region of the venous blood pathway adjacent to the distal end region of the cannula.
